# EUROPÄISCHE PATENTSCHRIFT

(11) **EP 1 632 198 B1**
(45) Veröffentlichungstag und Bekanntmachung des Hinweises auf die Patenterteilung: **02.11.2006**
(21) Anmeldenummer: 05014173.8
(22) Anmeldetag: 30.06.2005
(51) Int. Cl.: A61F 2/28, A61F 2/78

(54) **Subkutanes, intramuskuläres Lager für ein starres transkutanes Implantat**
Subcutaneous, intra-muscular coupling for a rigid transcutaneous implant
Logement intramusculaire sous-cutané pour implant rigide transcutané

(30) Priorität: 07.09.2004 DE 202004014043 U
(43) Veröffentlichungstag der Anmeldung: 08.03.2006
(73) Patentinhaber: ESKA Implants GmbH & Co. KG, 23556 Lübeck (DE)
(72) Erfinder: Grundei, Hans, Dr.-Ing., 23558 Lübeck (DE)
(74) Vertreter: Fuchs Mehler Weiss & Fritzsche

(56) Entgegenhaltungen:
- EP-A- 0 792 654
- EP-A- 1 378 215
- EP-A- 1 529 501
- DE-B3- 10 247 397
- US-A- 5 281 221

## Beschreibung

Die Erfindung betrifft ein subkutanes, intramuskuläres Lager für ein starres transkutanes Implantat, welches intrakorporal in einem Knochenstumpf verankerbar ist und welches ein Zwischenstück zwischen dem Implantat und einer daran ankoppelbaren extrakorporalen Kopplungseinrichtung aufweist.

Ein derartiges Lager ist bekannt aus der DE 100 40 590 A1. Das darin beschriebene Lager besteht aus einem flexiblen Material und es weist eine Tülle auf, die das Implantat distal fest umschließt, sowie eine intrakorporal anzuordnende Überwurfhülse in Form eines flexiblen Faltenbalges, der proximal mit einem angeformten Bund in abdichtender Weise mit der Tülle verbunden ist, derart, dass zwischen der Innenwandung des Faltenbalges und Außenwandung der Tülle ein Hohlraum einer Mindestbreite frei bleibt. Dabei ist distal am Faltenbalg ein flexibles Gittemetzwerk angeordnet, dem sich distalseitig ein weiteres Gittemetzwerk mit einem höheren E-Modul anschließt.

Mit diesem Lager wird das Ziel verfolgt, dass sich Weichteile gegenüber dem starren Implantat bewegen können, ohne dass die Durchbruchstelle im Körperstumpfteil einem erhöhten Risiko einer Entzündung ausgesetzt wird.

Wenn dieses bekannte Lager auch bereits in der Praxis erfolgreich eingesetzt wird, birgt es das Risiko, dass im Falle beispielsweise einer Reinigung der Durchtrittstelle des Implantates durch den Oberschenkelstumpf mit einer Kanüle durch das flexible Material, in den meisten Fällen Silikon, hindurchgestochen wird und eine Verkeimung stattfindet.

Um dem entgegenzuwirken, ist in der DE 102 47 397 B3 ein subkutanes, intramuskuläres Lager vorgeschlagen worden, das eine mit dem Zwischenstück fest verbundene starre Buchse derart aufweist, daß zwischen der Wandung der Buchse und dem Zwischenstück ein in Richtung intrakorporal geschlossener Ringraum ausgebildet ist, in den die extrakorporale Kopplungseinrichtung setzbar ist. Es weist einen auf die Außenwandung der Buchse aufgebrachten Schlauch aus flexiblem Material und auf den flexiblen Schlauch aufgebrachte metallische Wolle auf. Hiermit wird das Ziel verfolgt, daß die Sicherheit gegen eine Verkeimung der Durchtrittsstelle des Implantates und der angrenzenden Bereiche des Oberschenkelstumpfes deutlich erhöht wird und daß ein versehentliches Entfemen der Keimschranke verhindert wird.

Wenn die Sicherheit bei diesem Lager auch schon recht hoch angesiedelt ist, besteht der Wunsch, diese noch weiter zu erhöhen, um dem Patienten eine äußert unangenehme Prozedur im Falle einer Keimung zu ersparen.

Vor diesem Hintergrund ist es nun die Aufgabe der vorliegenden Erfindung, ein gattungsgemäßes, subkutanes intramuskuläres Lager der eingangs genannten Art so weiterzubilden, dass die Sicherheit gegen eine Verkeimung der Durchtrittsstelle des Implantates und der angrenzenden Bereiche des Oberschenkelstumpfes nochmals deutlich erhöht wird.

Demgemäß wird vorgeschlagen, dass das Lager auf der Außenwandung der Buchse eine offenmaschige, dreidimensionale Raumnetzstruktur aufweist, allerdings unter Aussparung im distalen Bereich um eine Breite B, vorzugsweise bis zu 2cm. Des Weiteren ist eine Hülse vorgesehen, die zwischen die Wandung der Buchse und die ankoppelbare Kopplungseinrichtung setzbar ist. Diese weist eine antibakteriell beschichtete Oberfläche auf.

Schließlich ist noch ein auf die Hülse setzbarer und dort verschieblich lagerbarer Ring mit ebenfalls einer antibakteriell beschichteter Oberfläche vorgesehen.

Die auf der Außenwandung der Buchse vorgesehene offenmaschige, dreidimensionale Raumnetzstruktur dient dazu, dass sich Bindegewebe darin einorganisieren kann und so eine Keimsperre ausbildet. Der distale Bereich bleibt dabei von der Raumnetzstruktur ausgespart, um so eine Bewegung des umgebenden Bindegewebes bei Ausgleichbewegungen zu ermöglichen.

Die Dimensionen sind so gewählt, daß zwischen der Wandung der Buchse und der Kopplungseinrichtung noch ein Spalt frei bleibt, in welchen die Hülse mit antibakteriell beschichteter Oberfläche geschoben werden kann. Sie ragt aus dem Oberschenkelstumpf heraus und bildet so die erste Barriere gegenüber einer etwaigen Verkeimung. Teil der Maßnahmen gegen eine Verkeimung ist darüber hinaus der auf die Hülse setzbare und dort verschieblich lagerbare Ring mit ebenfalls einer antibakteriell beschichteten Oberfläche.

Die antibakterielle Wirkung der Oberfläche der Hülse und des Ringes wird vorzugsweise realisiert durch eine Versilberung der Oberfläche. Bekanntermaßen wirkt Silber in der gewünschten Weise.

Gemäß einer vorteilhaften Weiterbildung weist der Ring einen umlaufenden Flansch auf. Der Ring ist nun so auf der Hülse verschiebbar, daß der Flansch so weit in Richtung auf den Oberschenkel verschoben werden kann, bis sich der Flansch an den Oberschenkelstumpf anlegt.

Operativ geht man nun so vor, dass nach der Amputation des Oberschenkels zunächst das transkutane Implantat im Knochenstumpf verankert wird und distalseitig mit dem Zwischenstück versehen wird. Daraufhin wird der Oberschenkelstumpf verschlossen, solange, bis das transkutane Implantat im Knochenstumpf verwachsen ist. Sodann, nach etwa 6 bis 8 Wochen, wird der Oberschenkelstumpf mit einer Hautstanze eröffnet. Dann wird die Hülse mit der antibakteriell beschichteten Oberfläche in den Ringraum gesetzt und der Ring mit der antibakteriell beschichteten Oberfläche über die Hülse geworfen und bis hin zur Durchtrittsstelle des Lagers durch den Oberschenkelstumpf geschoben. Sodann kann die extrakorporale Kopplungseinrichtung in den Ringraum geführt und dort befestigt werden.

Die Maschenweiten der dreidimensionalen Raumnetzstruktur auf der Buchse betragen vorzugsweise 50µm bis 2500µm. Diese Maschenweiten sind relativ groß, nehmen jedoch das sie umgebende Bindegewebe in hinreichendem Maße auf, damit eine sichere Keimsperre entstehen kann.

Die Erfindung wird anhand eines Ausführungsbeispiels näher erläutert. Hierbei zeigt:
- Fig. 1: das subkutane, intramuskuläre Lager, an welchem eine extrakorporale Kopplungseinrichtung angekoppelt ist,
- Fig. 2: den Ring mit antibakteriell beschichteter Oberfläche und
- Fig. 3: eine schematische Ansicht der Hülse mit antibakteriell beschichteter Oberfläche.

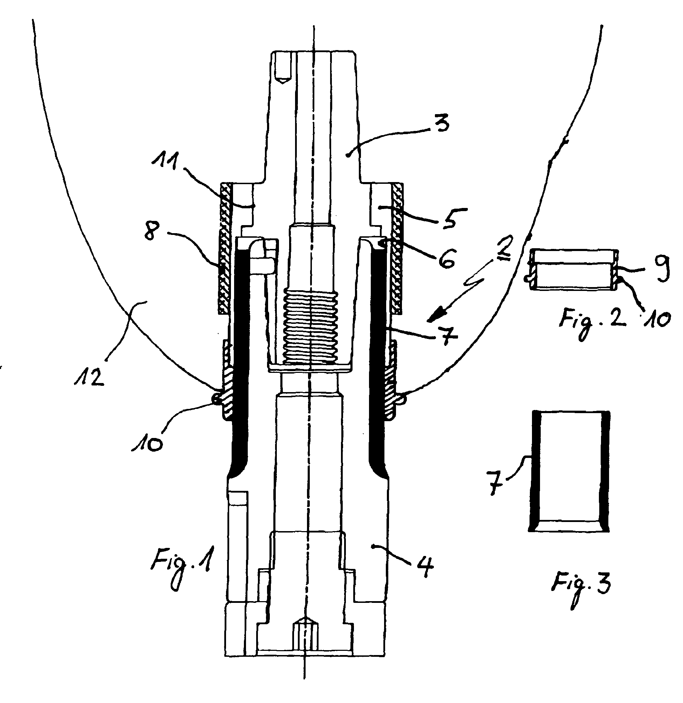

Das subkutane Implantat, welches vorliegend nicht dargestellt ist, wird an das Zwischenstück (3) gemäß Fig. 1 angekoppelt mittels einer konischen Verklemmung. Das Zwischenstück (3) ist vorliegend als Doppelkonus ausgebildet und weist einen zylindrischen Mittelabschnitt (11) auf, auf dem die Buchse (5) vorliegend aufgeschrumpft ist. Dem Mittelabschnitt (11) schließt sich distalseitig ein weiterer Konus zur Herstellung einer konischen Verklemmung mit einer konischen Klemmhülse in einem Adapter der extrakorporalen Koppelungseinrichtung (4).

Die Buchse ist so ausgebildet, daß zwischen ihrer Wandung und dem Zwischenstück (3) ein in Richtung intrakorporal oder proximal geschlossener Ringraum ausgebildet ist. In diesen Ringraum wird die antibakteriell beschichtete Hülse (7) gesetzt, ebenso wie die extrakorporale Kopplungseinrichtung (4). Die Hülse ist so dimensioniert, daß sie aus dem hier nur schemenhaft dargestellten Oberschenkelstumpf austritt. Bevor die Kopplungseinrichtung (4) in das Zwischenstück (3) geführt wird, wird der Ring (9) mit antibakteriell beschichteter Oberfläche auf die Hülse (7) gesetzt und soweit vorgeschoben in Richtung Oberschenkelstumpf, bis der am Ring (9) vorgesehene umlaufende Flansch (10) an der Durchtrittstelle im Oberschenkelstumpf zu liegen kommt.

Die Außenseite der Buchse (5) weist im proximalen Bereich eine offenmaschige, dreidimensionale Raumnetzstruktur (8) auf, in welche das nach Implantation der Buchse (5) umgebende Bindegewebe eingranuliert, um so eine Keimsperre auszubilden. Im distalen Bereich der Außenwandung der Buchse (5) befindet sich in einem Bereich der Breite B keine Raumnetzstruktur. Dies gestattet eine Ausgleichsbewegung des umgebenden Bindegewebes, ohne daß es zu Spannungen im Gewebe kommt.

### Bezugszeichenliste

- 2: subkutanes, intramuskuläres Lager
- 3: Zwischenstück
- 4: Extrakorporale Kopplungseinrichtung
- 5: Buchse
- 6: Ringraum
- 7: Hülse
- 8: Raumnetzstruktur
- 9: Ring
- 10: Flansch
- 11: Zylindrischer Mittelabschnitt
- 12: Oberschenkelstumpf

## Patentansprüche

1. Subkutanes, intramuskuläres Lager (1) für ein starres transkutanes Implantat (2), welches intrakorporal in einem Knochenstumpf verankerbar ist, das ein Zwischenstück (3) zwischen dem Implantat (2) und einer daran ankoppelbaren extrakorporalen Kopplungseinrichtung (4) aufweist, wobei eine mit dem Zwischenstück (3) fest verbundene starre Buchse (5) derart vorgesehen ist, dass zwischen der Wandung der Buchse (5) und dem Zwischenstück (3) ein in Richtung intrakorporal geschlossener Ringraum (6) ausgebildet ist, in den die extrakorporale Kopplungseinrichtung setzbar ist,
**gekennzeichnet durch**
- eine offenmaschige, dreidimensionale Raumnetzstruktur (8) auf der Außenwandung der Buchse (5) unter Aussparung im distalen Bereich um eine Breite B,
- eine Hülse (7), die zwischen die Wandung der Buchse (5) und die ankoppelbare Kopplungseinrichtung (4) setzbar ist, mit einer antibakteriell beschichteten Oberfläche, und
- ein auf die Hülse (7) setzbarer und dort verschieblich lagerbarer Ring (9) mit einer antibakteriell beschichteten Oberfläche.

2. Lager nach Anspruch 1, **dadurch gekennzeichnet, daß** die antibakteriell beschichteten Oberflächen realisiert sind durch Versilberung.

3. Lager nach Anspruch 1 oder 2, **dadurch gekennzeichnet, dass** der Ring einen umlaufenden Flansch (10) aufweist.

4. Lager nach einem der Ansprüche 1 bis 3, **dadurch gekennzeichnet, dass** der raumnetzstrukturfreie Bereich der Buchse (5) eine Breite B von bis zu 2 cm aufweist.

5. Lager nach einem der Ansprüche 1-4, **dadurch gekennzeichnet, dass** die Maschenweiten der Raumnetzstruktur im Bereich 500-2500 µm betragen.

## Claims

1. Subcutaneous, intramuscular bearing (1) for a rigid transcutaneous implant (2), which can be anchored intracorporeally in a bone stump and which has an intermediate piece (3) between the implant (2) and an extracorporeal coupling device (4) which can be coupled thereto, wherein a rigid bushing (5) firmly connected to the intermediate piece (3) is provided such that an annular gap (6) which is closed in the intracorporeal direction is formed between the wall of the bushing (5) and the intermediate piece (3), into which annular gap (6) the extracorporeal coupling device can be placed, **characterised by**
- an open-mesh, three-dimensional spatial network structure (8) on the outer wall of the bushing (5) with exception in the distal region by a width B,
- a sleeve (7), which can be placed between the wall of the bushing (5) and the coupling device (4) which can be coupled, with an anti-bacterially coated surface, and
- a ring (9), which can be placed on the sleeve (7) and can be mounted displaceably there, with an anti-bacterially coated surface.

2. Bearing according to claim 1, **characterised in that** the anti-bacterially coated surfaces are realised by silver coating.

3. Bearing according to claim 1 or 2, **characterised in that** the ring has an annular flange (10).

4. Bearing according to one of claims 1 to 3, **characterised in that** the region of the bushing (5) which is free of spatial network structure has a width B of up to 2 cm.

5. Bearing according to one of claims 1-4, **characterised in that** the mesh widths of the spatial network structure are in the range 500-2,500 µm.

## Revendications

1. Support sous-cutané intramusculaire (1) pour un implant transcutané rigide (2), qui peut être ancré par voie intracorporelle dans un moignon d'os et présente une partie intermédiaire (3) entre l'implant (2) et un dispositif de couplage (4) extracorporel qui peut lui être couplé, dans lequel il est prévu une douille rigide (5) raccordée fixement à la partie intermédiaire (3) de manière à former entre la paroi de la douille (5) et la partie intermédiaire (3) un espace annulaire fermé en direction intracorporelle (6), dans lequel on peut disposer le dispositif de couplage extracorporel, **caractérisé par**
- une structure réticulée tridimensionnelle à mailles ouvertes (8) sur la paroi externe de la douille (5) en formant dans la zone distale un évidement d'une largeur B,
- un manchon (7) que l'on peut placer entre la paroi de la douille (5) et le dispositif de couplage (4) qui peut lui être couplé, doté d'une surface à revêtement antibactérien, et
- un anneau (9) que l'on peut placer et que l'on peut déplacer sur le manchon (7), doté d'une surface à revêtement antibactérien.

2. Support selon la revendication 1, **caractérisé en ce que** les surfaces à revêtement antibactérien sont réalisées par argentage.

3. Support selon la revendication 1 ou 2, **caractérisé en ce que** l'anneau présente une collerette périphérique (10).

4. Support selon l'une quelconque des revendications 1 à 3, **caractérisé en ce que** la zone de la douille (5) sans structure réticulée présente une largeur B allant jusqu'à 2 cm.

5. Support selon l'une quelconque des revendications 1 à 4, **caractérisé en ce que** les largeurs de mailles de la structure réticulée se situent dans la plage de 500 à 2500 µm.
